(19) Europäisches Patentamt
European Patent Office
Office européen des brevets

(11) Publication number: **0 446 373 A1**

(12) **EUROPEAN PATENT APPLICATION published in accordance with Art. 158(3) EPC**

(21) Application number: **90914419.8**

(22) Date of filing: **28.09.90**

(86) International application number: **PCT/JP90/01251**

(87) International publication number: **WO 91/04981 (18.04.91 91/09)**

(51) Int. Cl.[5]: **C07H 19/052**, C07H 19/16, C07H 19/23, C07D 305/08

(30) Priority: **29.09.89 JP 251997/89**

(43) Date of publication of application: **18.09.91 Bulletin 91/38**

(84) Designated Contracting States: **DE FR GB**

(71) Applicant: **NIPPON KAYAKU KABUSHIKI KAISHA**
**11-2, Fujimi 1-chome Chiyoda-ku**
**Tokyo 102(JP)**

(72) Inventor: **TAKETSURU, Hirofumi**
**5-13-23, Shimoochiai**
**Yono-shi Saitama 338(JP)**
Inventor: **KOGAWA, Osamu**
**1072-14, Takada**
**Kashiwa-shi Chiba 277(JP)**

(74) Representative: **Türk, Dietmar, Dr. rer. nat.**
**Türk, Gille + Hrabal Patentanwälte**
**Brucknerstrasse 20**
**W-4000 Düsseldorf 13(DE)**

(54) **NOVEL METHOD OF PRODUCING OXETANOCIN DERIVATIVE.**

(57) A method of producing an oxetanocin derivative of formula (III) by the condensation of an oxetane compound of formula (I) with either a pyrimidine compound having protected functional group(s) or a condensed imidazole compound of formula (II). The reaction is conducted in an organic solvent such as a haloalkane or an aromatic hydrocarbon or without using any solvent, at a temperature of ordinarily -40 to 200 °C, optionally in the presence of a salt of a metal such as tin, mercury, silver, sodium or lithium and further optionalluy in the presence of an additive such as molecular sieve.

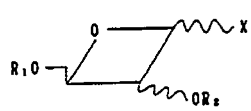
(I)

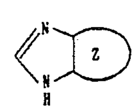
(II)

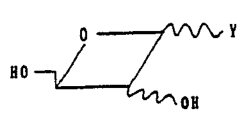
(III)

EP 0 446 373 A1

[TECHNICAL FIELD]

This invention relates to a novel process for synthesizing oxetanocin derivatives having activities such as antiviral action and the like.

[BACKGROUND ART]

Oxetanocin is a novel antibiotic substance discovered by Shimada et al. [The Journal of Antibiotics, Vol. XXXIX, No. 11, Pages 1623-1625 (1986)], and its derivatives are synthesized from oxetanocin (ibid. Vol. XL, No. 12, Pages 1788-1790 (1987)).

[PROBLEM THAT THE INVENTION INTENDS TO SOLVE]

When oxetanocin derivatives are derived from oxetanocin, the derivatives which can be produced are inevitably limited. Therefore, development of a synthetic production process using no oxetanocin as starting material is waited for.

[DISCLOSURE OF THE INVENTION]

The present inventors studied the synthesis of oxetanocin derivatives in various manners. As a result of the studies, this invention was accomplished.

That is, this invention relates to a novel process for producing an oxetanocin derivative represented by the following formula:

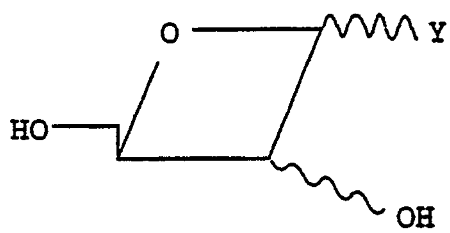

(III)

wherein Y is a pyrimidine compound residue (when an amino group is introduced, Y is a pyrimidine compound residue into which an amino group has been introduced) bonding to the oxetane ring at the nitrogen atom, or a condensed imidazole compound residue represented by the following formula:

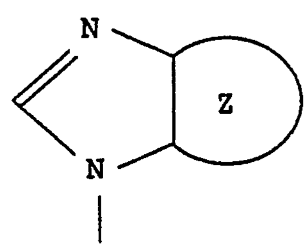

(Z represents a condensed 6-membered ring containing a nitrogen atom bonding to the imidazole ring), characterized by reacting an oxetane compound represented by the following formula:

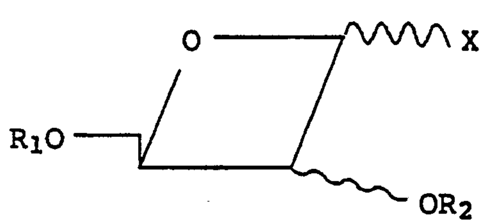

(I)

wherein $R_1$ and $R_2$ represent a protecting group and X represents a halogen atom, with

(i) a pyrimidine compound of which functional group is protected or

(ii) a condensed imidazole compound represented by the following formula:

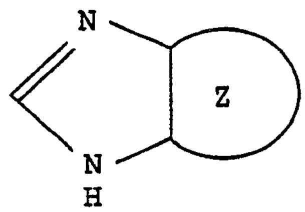

(II)

wherein Z represents a condensed 6-membered ring containing a nitrogen atom bonding to the imidazole ring and the functional group on the condensed ring may be protected if necessary, and then introducing an amino group into the pyrimidine ring if necessary, and then removing the protecting groups if necessary, and also relates to an oxetane compound represented by the formula (I).

The protecting groups $R_1$ and $R_2$ in formula (I) are not particularly limited, and they may be either separated or bonded to each other. As $R_1$ and $R_2$, acyl type, benzyl type and silyl type protecting groups and the like conventionally used as protecting group of hydroxy group can be referred to. Generally speaking, however, protecting groups eliminable by reduction are preferable, and benzyl type protecting groups can be referred to as example of such preferable protecting group. As X, halogen atoms such as chlorine atom, bromine atom and the like can be referred to.

As the pyrimidine compound of which functional group is protected, cytosines of which functional group is protected and uracils of which protecting group is protected can be referred to. Such uracils may be any of (1) uracil and (2) uracils having a substituent. As the substituent, lower alkyl group, alkenyl group, lower alkoxy group, halogen atom, halogen-substituted lower alkyl group, halogen-substituted alkenyl group and the like can be referred to.

As the condensed 6-membered ring Z of the formula (II), those having 1-3 nitrogen atoms can be referred to usually.

As the condensed imidazole of formula (II), purine bases can be referred to generally.

As the pyrimidine compound residue of general formula (III), cytosines and substituted or unsubstituted uracil residues can be referred to.

[BEST MODE FOR CARRYING OUT THE INVENTION]

In the next place, the synthetic process of this invention will be explained.

The condensation reaction of the first step between the compound of formula (I) and the pyrimidine compound or condensed imidazole compound of which functional group is protected is carried out either in an organic solvent or in the absence of solvent, usually at a temperature of about -40 ° C to about 200 ° C and preferably at about 0 ° C to about 150 ° C, in the presence or absence of a metallic salt, in the presence or absence of an additive such as 18-crown-6, molecular sieves or the like. As the organic solvent, halogeno-alkanes such as dichloromethane, dichloroethane and the like; aromatic hydrocarbons such as benzene, toluene, xylene, nitrobenzene, dichlorobenzene and the like; acetonitrile; dimethoxyethane; acetone; nitromethane; nitroethane; dimethylformamide; dimethyl sulfoxide; and the like are used either singly or in a mixed form. As the metallic salt, for example, tin salts (tin tetra-chloride, tin tetrabromide and the like), mercury salts (mercurous chloride, mercuric chloride, mercurous cyanide, mercuric cyanide, mercuric bromide, mercuric iodide, mercuric oxide and the like), silver salts (silver perchlorate and the like), potassium salts (potassium carbonate, potassium hydroxide, potassium hydride and the like), sodium salts (sodium carbonate, sodium hydroxide, sodium hydride and the like), lithium salts (lithium hydride and the like), etc are used.

In introducing an amino group into the pyrimidine ring, the general method of converting a protected hydroxy group into an amino group is used. For example, in an organic solvent miscible with water (lower alcohol such as methanol, ethanol and the like; tetrahydrofuran; dioxane; dimethylformamide; dimethyl sulfoxide; and the like), a reaction with aqueous ammonia solution or a lower amine is carried out usually at 0 ° C to 150 ° C and preferably at 50 ° C to 100 ° C.

The removal of protecting group is carried out according to the general method using an acid or a base which eliminates the used protecting group without exercising substantial influence upon the oxetane ring. For removing a silyl type protecting group, fluorine compounds can also be used. For removing a benzyl

type protecting group, reductive methods such as a catalytic reduction using a solid catalyst such as palladium-black, palladium supported on active charcoal and the like in an inert solvent in the presence of hydrogen or a hydrogen donor such as formic acid, cyclohexene, cyclohexadiene or the like can be used.

Next, this invention will be explained more concretely with reference to examples.

[Example 1]

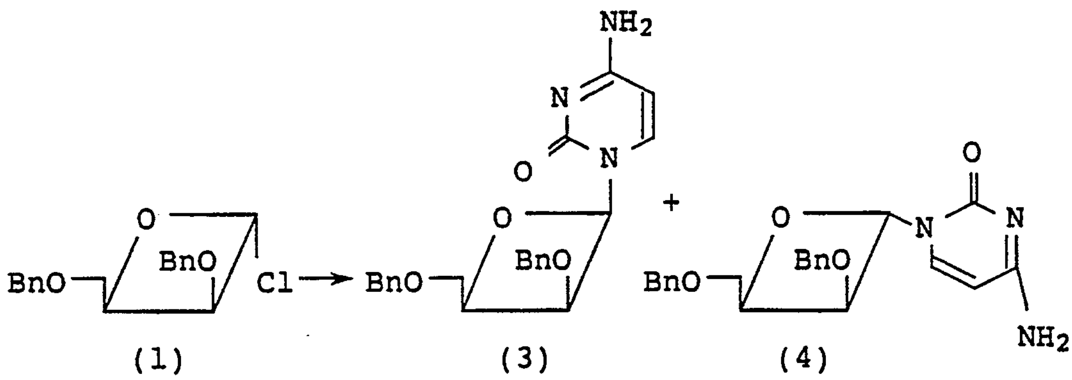

(1) (3) (4)

(Bn = 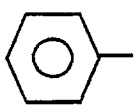 $CH_2$ : Hereinafter the same)

Syntheses of 2',4'-di-O-benzyl-β-D-threooxetanosylcytosine (Compound (3)) and 2',4'-di-O-benzyl-α-D-threooxetanosylcytosine (Compound (4))

Compound (1) (25.3 mg, 0.079 mmole) was dissolved in acetonitrile (1 ml). 2,4-Dimethoxy-pyrimidine (25.0 mg, 0.179 mmole) and mercuric bromide (32.0 mg, 0.089 mmole) were added thereto, and the resulting mixture was stirred for 15 hours at room temperature. Then, 30% aqueous solution of potassium iodide was added to the reaction mixture and stirred for 10 minutes, and then extraction with chloroform was carried out three times. The chloroform extract solution was washed with saturated aqueous solution of sodium chloride, dried over sodium sulfate and concentrated under reduced pressure. The residue thus obtained was dissolved in dioxane (1.5 ml), thereto was added concentrated aqueous ammonia (0.75 ml), and the mixture was reacted in an ampoule at 100°C for 14 hours. The solvent was distilled off, and the residue was separated by means of preparative silica gel thin layer chromatography (chloroform:methanol = 10:1), eluted with methanol, and purified by Sephadex® LH-20 column chromatography. Thus, Compound (3) (5.4 mg, yield 17%) and Compound (4) (12.1 mg, yield 39%) were obtained in the form of colorless powder.

Compound (3)

Rf 0.45 (silica gel thin layer chromatography Art. 5715, manufactured by Merck Co.; developing solvent chloroform:methanol = 9:1)
$^1$H-NMR (200 MHz, $CD_3OD$): 3.81 (2H, ddd, J=5.2, 6.1 & 11.4) 4.33 (1H, d, J=11.5) 4.40 (1H, d, J=11.5) 4.54 (2H, brs) 4.83 (1H, dd, J=4.4 & 5.8) 5.08 (1H, ddd, J=5.2, 5.6 & 6.1) 5.77 (1H, d, J=7.5) 6.57 (1H, d, J=4.4) 7.09-7.39 (10H, m) 7.88 (1H, d, J=7.5)

Compound (4)

Rf 0.40 (silica gel thin layer chromatography Art. 5715, manufactured by Merck Co.; developing solvent chloroform:methanol = 9:1)
$^1$H-NMR (200 MHz, $CD_3OD$): 3.89 (2H, d, J=5.5) 4.48-4.71 (4H, m) 4.64 (1H, dd, J=4.4 & 7.1) 4.95 (1H, dt, J=5.5 & 7.1) 5.93 (1H, d, J=7.5) 6.32 (1H, d, J=4.4) 7.17-7.41 (10H, m) 7.79 (1H, d, J=7.5)

[Example 2]

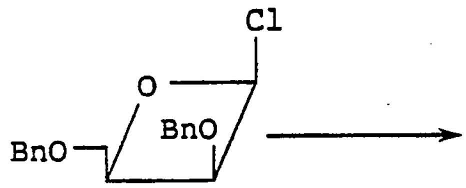

(3) + (4)

(2)

Syntheses of 2',4'-di-O-benzyl-β-D-threooxetanosyl-cytosine (Compound (3)) and 2',4'-di-O-benzyl-α-D-threooxetanosylcytosine (Compound (4))

Compound (2) (29.3 mg, 0.092 mmole) was dissolved in acetonitrile (1 ml). 2,4-Dimethoxy-pyrimidine (25.0 mg, 0.179 mmole) and mercuric bromide (41.3 mg, 0.114 mmole) were added thereto and stirred at room temperature for 16 hours. Then, the mixture was post-treated in the same manner as in Example 1. The residue thus obtained was dissolved in dioxane (1.3 ml), concentrated aqueous ammonia (0.5 ml) was added thereto, and the mixture was reacted in an ampoule at 95°C for 15 hours. The product was purified in the same manner as in Example 1 to obtain Compound (3) (2.5 mg, yield 7%) and Compound (4) (17.9 mg, yield 50%).

[Example 3]

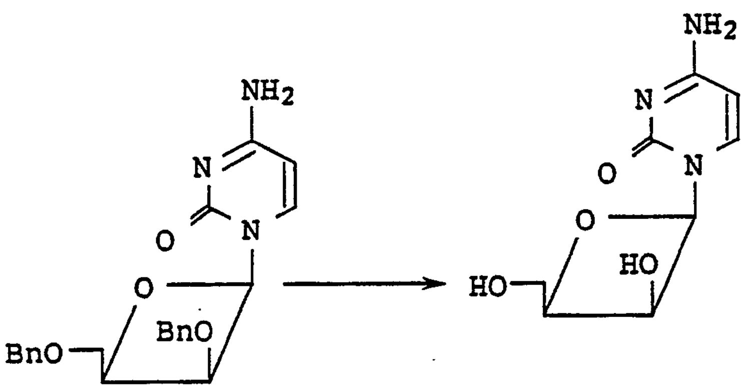

(3) (5)

Synthesis of β-D-threooxetanosylcytosine (Compound (5))

Palladium-black (39.3 mg) was added to 8% methanolic solution of formic acid (2 ml) and stirred for one minute. Subsequently, Compound (3) (14.0 mg, 0.036 mmole) dissolved in methanol (2 ml) was added thereto. The resulting mixture was stirred at room temperature for 7.5 hours and filtered, and the filtrate was concentrated under reduced pressure. From the residue thus obtained, the objective compound was isolated by preparative silica gel thin layer chromatography (chloroform:methanol:water = 120:50:3). It was dissolved in methanol and purified by Sephadex® H-20 column chromatography to obtain Compound (5) (5.6 mg, yield 74%) in the form of colorless powder.

Compound (5)

Rf 0.36 (silica gel thin layer chromatography Art. 5715, manufactured by Merck Co.; developing solvent chloroform:methanol:water = 60:20:1)
$^1$H-NMR (200 MHz, DMSO-$d_6$): 3.68 (2H, brs) 4.62-4.85 (3H, m) 5.76 (2H, d, J=7.4) 6.40 (1H, d, J=4.4) 7.01-7.33 (2H) 7.78 (1H, d, J=7.4)

[Example 4]

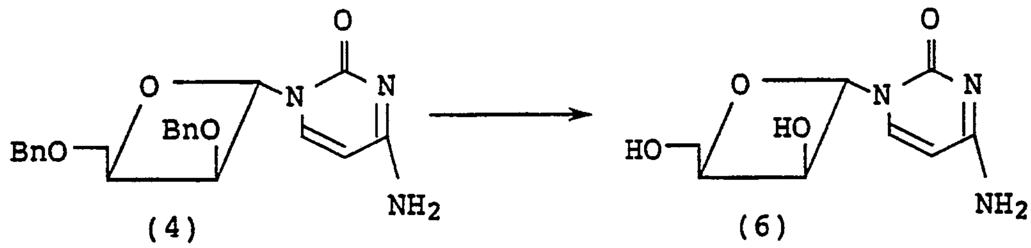

(4) (6)

Synthesis of α-D-threooxetanosylcytosine (Compound (6))

Palladium-black (21.6 mg) was added to 8% methanolic solution of formic acid (2 ml) and stirred for one minute. Subsequently, Compound (4) (15.0 mg, 0.038 mmole) dissolved in methanol (2 ml) was added thereto. The resulting mixture was stirred at room temperature for 3 hours and filtered, and then the filtrate was concentrated under reduced pressure. From the residue thus obtained, the objective compound was isolated by preparative silica gel thin layer chromatography (chloroform:methanol:water = 120:50:3). It was dissolved in methanol and purified by Sephadex® LH-20 column chromatography to obtain Compound (6) (4.5 mg, yield 55%) in the form of colorless powder.

Compound (6)

Rf 0.25 (silica gel thin layer chromatography Art. 5715, manufactured by Merck Co.; developing solvent chloroform:methanol:water = 60:20:1)
$^1$H-NMR (200 MHz, DMSO-$d_6$): 3.75 (2H, brs) 4.58 (1H, m) 4.65-4.86 (2H, m) 5.81 (1H, d, J=7.4) 5.87 (1H, d, J=6.4) 6.28 (1H, d, J=4.8) 7.18-7.40 (2H) 7.86 (1H, d, J=7.4)

[Example 5]

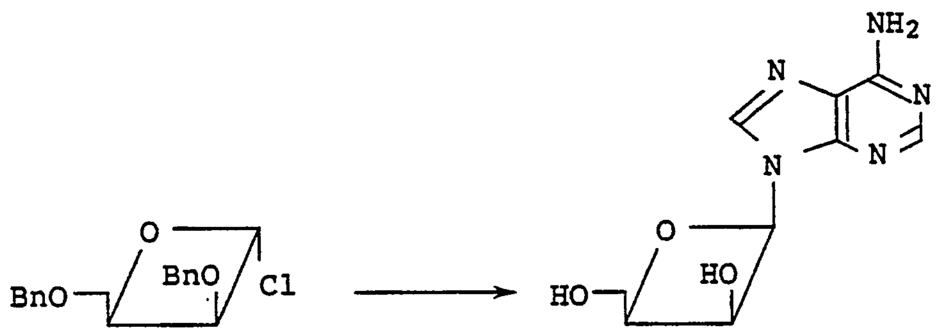

(1) (7)

Synthesis of β-D-threooxetanosyladenine (Compound (7))

Compound (1) (30.3 mg, 0.095 mmole) was dissolved in a solvent mixture consisting of acetonitrile (1 ml) and dimethylformamide (1 ml). After adding adenine (38.5 mg, 0.285 mmole), anhydrous potassium carbonate (26.0 mg, 0.188 mmole) and 18-crown-6 (49.8 mg, 0.189 mmole), the resulting mixture was stirred at 100°C for 5 hours. Silica gel (200 mg) was added thereto and the solvent was distilled off under reduced pressure. The residue was charged onto a silica gel column having been equilibrated with chloroform and eluted with chloroform:methanol (9:1) mixture. The fractions containing the objective compound were collected, and the solvent was distilled off under reduced pressure. The residue thus obtained was subjected to elimination of protecting group, isolation and purification under the same conditions as in Example 3. Thus, Compound (7) (3.4 mg, yield 15%) was obtained in the form of colorless

powder.

Compound (7)

Rf 0.35 (silica gel thin layer chromatography Art. 5715, manufactured by Merck Co.; developing solvent chloroform:methanol = 20:1)
$^1H$-NMR (400 MHz, $D_2O$): 3.93-4.08 (2H, m) 5.07 (1H, m) 5.21 (1H, m) 6.78 (1H, d, J=5.2) 8.15 (1H, s) 8.53 (1H, s)

Referential Example

Compound (1) and Compound (2) used in this invention as starting material are novel compounds, and they are synthesized in the following manner. Thus, they are synthesized by applying the known methods (G.W.J. Fleet et al., Tetrahedron Lett., 28, 4741 ('87); ibid., 29, 1449 ('88)) of ring contraction and halogenation to the corresponding protected lactones.

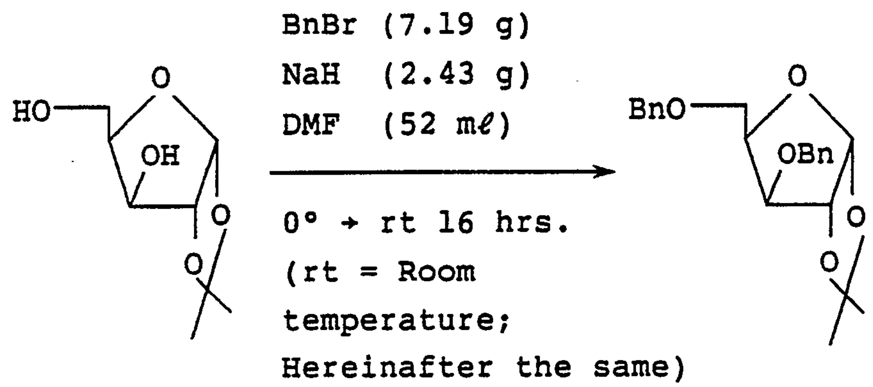


1,2-O-Isopropylidene-D-xylofuranose (Commercial product)

(Amount: 2.01 g)

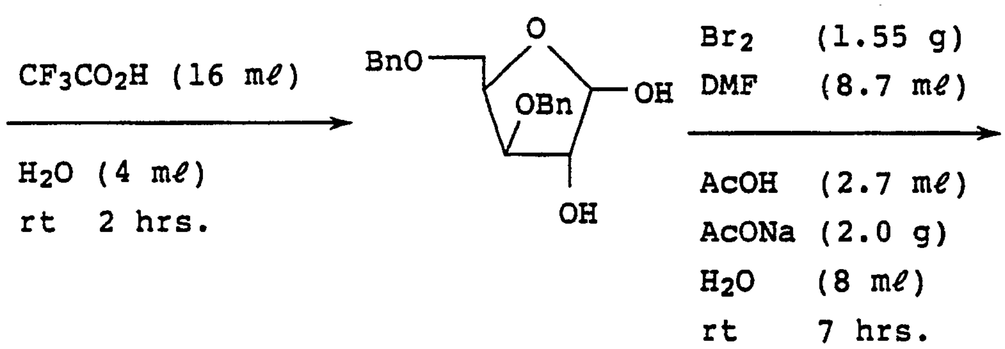

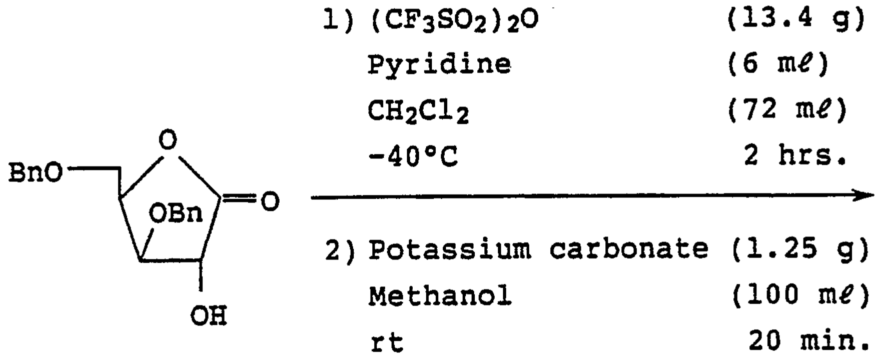


(y: 72%, based on 1,2-O-isopropylidene-D-xylofuranose)

(Amount used: 2.16 g)

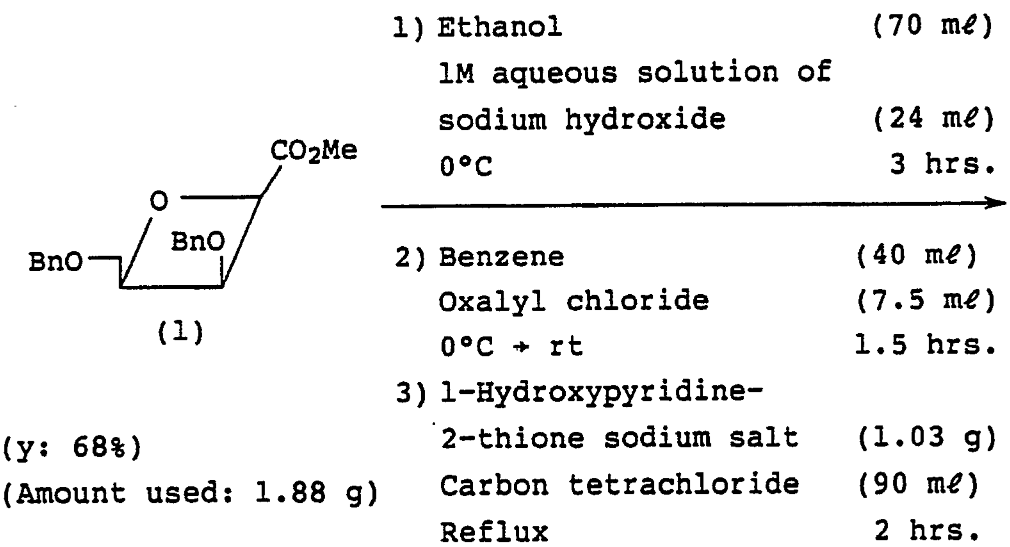


(1)

(y: 68%)

(Amount used: 1.88 g)

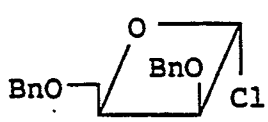

(1)

(y: 19%)

+

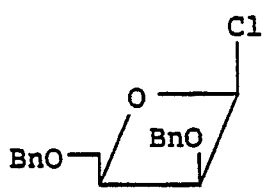

(2)

(y: 19%)

## Claims

1. A novel process for producing an oxetanocin derivative represented by the following formula (III):

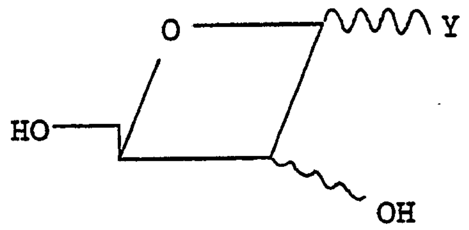

(III)

wherein Y represents a pyrimidine compound residue (when an amino group is introduced, Y represents a pyrimidine compound residue into which an amino group has been introduced) bonding to oxetane ring at the nitrogen atom, or a condensed imidazole compound residue represented by the following formula:

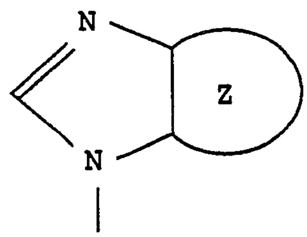

(Z represents a condensed 6-membered ring containing a nitrogen atom bonding to imidazole ring), characterized by reacting an oxetane compound represented by the following formula:

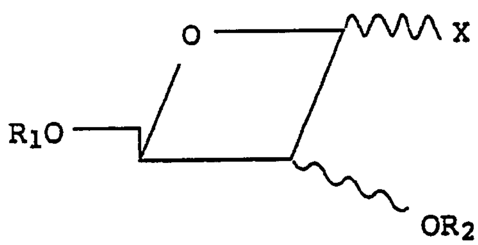

(I)

wherein $R_1$ and $R_2$ each represent a protecting group and X represents a halogen atom, with

(i) a pyrimidine compound of which functional group is protected, or

(ii) a condensed imidazole compound represented by the following formula:

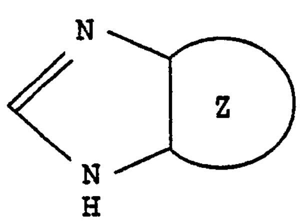

(II)

wherein Z represents a condensed 6-membered ring containing a nitrogen atom bonding to imidazole ring and the functional group on the condensed ring is protected if necessary) and subsequently, if necessary, introducing an amino group into the pyrimidine ring and subsequently, if necessary, eliminating the protecting group.

**2.** An oxetane compound represented by the following formula:

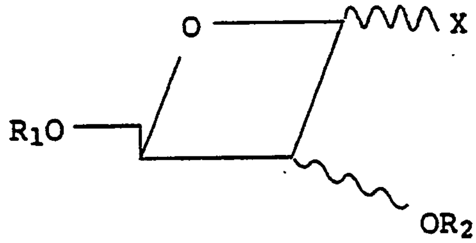

(I)

wherein $R_1$ and $R_2$ each represent a protecting group and X represents a halogen atom.

**Amended Claims**

1. A novel process for producing an oxetanocin derivative represented by the following formula (III):

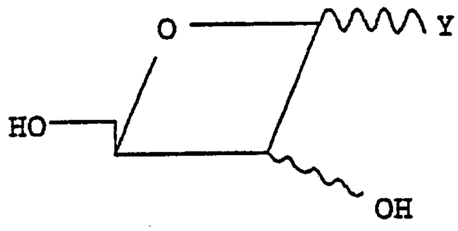

(III)

wherein Y represents a pyrimidine compound residue (when an amino group is introduced, Y represents a pyrimidine compound residue into which an amino group has been introduced) bonding to oxetane ring at the nitrogen atom, or a condensed imidazole compound residue represented by the following formula:

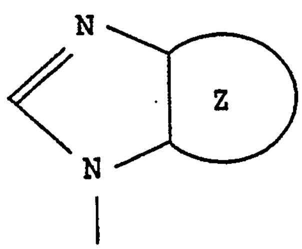

(Z represents a condensed 6-membered ring containing a nitrogen atom bonding to imidazole ring), characterized by reacting an oxetane compound represented by the following formula:

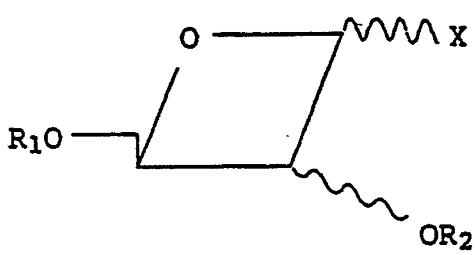

(I)

wherein $R_1$ and $R_2$ each represent a protecting group and X represents a halogen atom, with
(i) a pyrimidine compound of which functional group is protected, or
(ii) a condensed imidazole compound represented by the following formula:

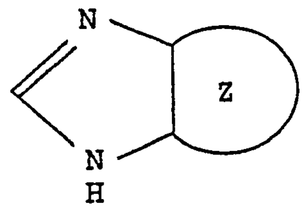

(II)

wherein Z represents a condensed 6-membered ring containing a nitrogen atom bonding to imidazole ring and the functional group on the condensed ring is protected if necessary) and subsequently, if necessary, introducing an amino group into the pyrimidine ring and subsequently, if necessary, eliminating the protecting group.

2. (Addition) A process for producing an oxetanocin derivative according to Claim 1, wherein, in formula (I), the protecting groups $R_1$ and $R_2$ are benzyl group and the halogen atom X is chlorine atom or bromine atom.

3. (Addition) A process for producing an oxetanocin derivative according to Claim 1, wherein said pyrimidine compound is cytosine, or substituted or unsubstituted uracil.

4. (Addition) A process for producing an oxetanocin derivative according to Claim 1, wherein said condensed imidazole compound is a purine base.

5. (Addition) A process for producing an oxetanocin derivative according to Claim 1, wherein said condensed imidazole compound is adenine.

6. (Addition) A process for producing an oxetanocin derivative according to Claim 1, wherein the condensation reaction is carried out in an organic solvent or in the absence of solvent, at a reaction temperature not exceeding 200°C.

7. (The original Claim 2)
An oxetane compound represented by the following formula:

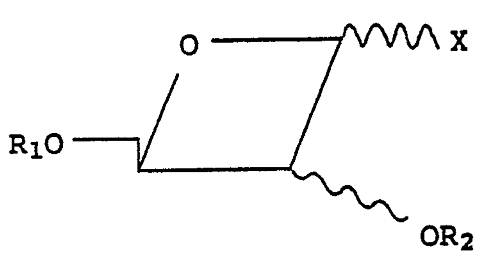

(I)

wherein $R_1$ and $R_2$ each represent a protecting group and X represents a halogen atom.

8. (Addition) An oxetane compound according to Claim 7, wherein, in formula (I), the protecting groups $R_1$ and $R_2$ are benzyl group and the halogen atom X is chlorine atom or bromine atom.

# INTERNATIONAL SEARCH REPORT

International Application No PCT/JP90/01251

**I. CLASSIFICATION OF SUBJECT MATTER** (if several classification symbols apply, indicate all) [6]

According to International Patent Classification (IPC) or to both National Classification and IPC

Int. $Cl^5$ C07H19/052, 19/16, 19/23, C07D305/08

**II. FIELDS SEARCHED**

Minimum Documentation Searched [7]

| Classification System | Classification Symbols |
|---|---|
| IPC | C07D305/08, C07H19/04-19/23 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | EP, A2, 334250 (NIPPON KAYAKU CO., LTD.), 27 September 1989 (27. 09. 89), (Family: none) | 1 - 2 |
| P | JP, A, 1-249792 (Nippon Kayaku Co., Ltd.), 5 October 1989 (05. 10. 89), (Family: none) | 1 - 2 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| December 4, 1990 (04. 12. 90) | December 17, 1990 (17. 12. 90) |
| **International Searching Authority** | **Signature of Authorized Officer** |
| Japanese Patent Office | |